# EUROPEAN PATENT SPECIFICATION

(11) **EP 3 129 047 B1**
(45) Date of publication and mention of the grant of the patent: **21.10.2020**
(21) Application number: 15776984.5
(22) Date of filing: 07.04.2015
(51) Int. Cl.: A61K 39/00, C07K 16/28

(54) **STABLE FORMULATIONS FOR ANTI-CD19 ANTIBODIES AND ANTIBODY-DRUG CONJUGATES**
STABILE FORMULIERUNGEN FÜR ANTI-CD19-ANTIKÖRPER UND ANTIKÖRPER-WIRKSTOFF-KONJUGATE
FORMULATIONS STABLES POUR DES ANTICORPS ANTI-CD19 ET CONJUGUÉS ANTICORPS-MÉDICAMENT

(30) Priority: 07.04.2014 US 201461976313 P
(43) Date of publication of application: 15.02.2017
(73) Proprietor: Seattle Genetics, Inc., Bothell, WA 98021 (US)
(72) Inventor: AMSBERRY, Kent, Bothell, Washington 98021 (US); JIANG, Shan, Bothell, Washington 98021 (US)
(74) Representative: Forresters IP LLP
(86) International application number: PCT/US2015/024719
(87) International publication number: WO 2015/157286

(56) References cited:
- WO-A2-2009/052431
- US-A1- 2012 148 576
- US-A1- 2013 209 496
- US-B1- 6 252 055
- WANG W ET AL: "ANTIBODY STRUCTURE, INSTABILITY, AND FORMULATION", JOURNAL OF PHARMACEUTICAL SCIENCES, AMERICAN PHARMACEUTICAL ASSOCIATION, WASHINGTON, US, vol. 96, no. 1, 1 January 2007 (2007-01-01), pages 1-26, XP009084505, ISSN: 0022-3549, DOI: 10.1002/JPS.20727
- NICHOLAS W WARNE: "Development of high concentration protein biopharmaceuticals: The use of platform approaches in formulation development", EUROPEAN JOURNAL OF PHARMACEUTICS AND BIOPHARMACEUTICS, ELSEVIER SCIENCE PUBLISHERS B.V., AMSTERDAM, NL, vol. 78, no. 2, 3 March 2011 (2011-03-03), pages 208-212, XP028203394, ISSN: 0939-6411, DOI: 10.1016/J.EJPB.2011.03.004 [retrieved on 2011-03-13]
- WANG WEI ED - BLANCO-PRIETO MARIA J ET AL: "Instability, stabilization, and formulation of liquid protein pharmaceuticals", INTERNATIONAL JOURNAL OF PHARMACEUTICS, ELSEVIER, NL, vol. 185, no. 2, 20 August 1999 (1999-08-20), pages 129-188, XP002323952, ISSN: 0378-5173, DOI: 10.1016/S0378-5173(99)00152-0
- Lene Jorgensen ET AL: "Recent trends in stabilising peptides and proteins in pharmaceutical formulation - considerations in the choice of excipients", Expert Opinion on Drug Delivery, vol. 6, no. 11, 13 August 2009 (2009-08-13), pages 1219-1230, XP55528446, GB ISSN: 1742-5247, DOI: 10.1517/17425240903199143

## Description

### FIELD OF THE INVENTION

This disclosure provides optimized formulations for CD19 antibodies and antibody-drug conjugates (ADCs).

### BACKGROUND OF THE INVENTION

CD19 is a member of the immunoglobulin superfamily. *See, e.g.,* Tedder & Isaacs, J Immunol, 143:712-717 (1989) and Del Nagro et al., Immunol Res, 31:119-131 (2005). It is a B cell-specific marker not known to be expressed by any cell outside of the B lineage. CD19 expression is maintained upon malignant transformation, thus, CD19 is found on malignant cells in the majority of patients with B-cell leukemia or non-Hodgkin lymphoma. *See, e.g.,* Nadler et al., J Immunol, 131:244-250 (1983); Anderson et al., Blood, 63:1424-1433 (1984); and Scheuermann & Racila, Leuk Lymphoma, 18:385-397 (1995).

SGN-CD19A is a CD19-directed antibody-drug conjugate (ADC) consisting of three components: 1) the humanized antibody hBU12, which specifically binds the human CD19 protein, 2) the microtubule disrupting agent, monomethyl auristatin F (MMAF), and 3) a stable linker, maleimidocaproyl, that covalently attaches MMAF to hBU12. The proposed mechanism of action (MOA) is initiated by SGN-CD19A binding to CD19 on the cell surface followed by internalization of the ADC. Upon trafficking to lysosomes, the delivered drug (cysmcMMAF) is released through proteolytic degradation of the antibody carrier. Binding of the released drug to tubulin disrupts the microtubule network, leading to cell cycle arrest and apoptosis. WO 2009/052431 and US 2012/148579 disclose anti-CD 19 antibodies. Wang et al., J.Pharm. Sci., v96, no.1, pages 1-26, 2017 and Warne, Euro.J. Pharmaceutics And Biopharmaceutics, v78, no.2, pages 208-212, 2011 disclose aspects of formulation of antibodies.

There is a need for SGN-CD19A formulations that provide stability of the molecule and thus, allow for transport and storage of the drug after manufacture. The present disclosure addresses these and other needs.

### BRIEF SUMMARY OF THE INVENTION

The invention is defined by the claims.

This disclosure provides stable formulations of an anti-CD 19 antibody including a phosphate buffer. The anti-CD 19 antibody has a light chain variable region of SEQ ID NO: 1 and a heavy chain variable region of SEQ ID NO:2, and the phosphate buffer has a pH value between 5.0 and 7.0. The phosphate buffer diminishes the occurance of oxidation of the antibody in the presence of light. In one embodiment, the phosphate buffer is a sodium phosphate buffer. In another embodiment, the phosphate buffer is a potassium phosphate buffer. In preferred embodiments, the phosphate buffer has a pH between 5.5 and 6.5. In a further embodiment, the anti-CD 19 antibody formulation includes a phosphate buffer having a pH of about 6.0. In another embodiment, the phosphate buffer of the formulation has a pH of 6.0.

In one embodiment, this disclosure provides an anti-CD 19 antibody with a light chain variable region of SEQ ID NO:1 and a heavy chain variable region of SEQ ID NO:2 in a stable formulation that includes a phosphate buffer. The anti-CD 19 antibody is conjugated to a cytotoxic agent. The cytotoxic agent is monomethylauristatin F (MMAF). The MMAF can be conjugated to the antibody via a maleimidocaproyl (mc) linker.

In one embodiment, this disclosure provides an anti-CD 19 antibody with a light chain variable region of SEQ ID NO:1 and a heavy chain variable region of SEQ ID NO:2 in a stable formulation that includes a phosphate buffer and polysorbate 80. The polysorbate 80 concentration is 0.02%.

In one embodiment, this disclosure provides an anti-CD 19 antibody with a light chain variable region of SEQ ID NO:1 and a heavy chain variable region of SEQ ID NO:2 in a stable formulation that includes a phosphate buffer and a bulking agent, e.g., a sugar. The sugar is sucrose and the sucrose concentration is 60 mg/ml.

In one embodiment, this disclosure provides an anti-CD 19 antibody with a light chain variable region of SEQ ID NO:1 and a heavy chain variable region of SEQ ID NO:2 in a stable formulation that includes a potassium phosphate at about pH 6.0; about 0.02% polysorbate 80, and about 60 mg/ml sucrose. The anti-CD19 antibody is conjugated to the drug-linker mcMMAF. The stable formulation of the anti-CD 19 antibody can be lyophilized before storage. A vial can be used for stage of the stable lyophilized the anti-CD 19 antibody formulation. The lyophilized the anti-CD 19 antibody formulation is then reconstituted in a sterile liquid before adminstration to a ptient. In further embodiments, the lyophilized formulation is stable for up to eighteen months at 25°C. In another embodiment, the stable formulation of the anti-CD19 antibody is a liquid formulation and is stored in the liquid state. The liquid fomulation is stored in a vial in an amount appropriate for adminstration to a patient. In further embodiments, the liquid formulation is stable for up to eighteen months at 5°C.

In one embodiment, this disclosure provides an anti-CD19 antibody with a light chain variable region of SEQ ID NO:1 and a heavy chain variable region of SEQ ID NO:2 in a stable formulation that includes a potassium phosphate at pH 6.0; 0.02% polysorbate 80, and 60 mg/ml sucrose. The anti-CD19 antibody is conjugated to the drug-linker mcMMAF. The stable formulation of the anti-CD 19 antibody can be lyophilized before storage. A vial can be used for stage of the stable lyophilized the anti-CD19 antibody formulation. The lyophilized the anti-CD19 antibody formulation is then reconstituted in a sterile liquid before adminstration to a ptient.. In further embodiments, the lyophilized formulation is stable for up to eighteen months at 25°C. In another embodiment, the stable formulation of the anti-CD19 antibody is a liquid formulation and is stored in the liquid state. The liquid fomulation is stored in a vial in an amount appropriate for adminstration to a patient. In further embodiments, the liquid formulation is stable for up to eighteen months at 5°C.

### DEFINITIONS

The term "stabilized," or "stable" in the context of isolated antibody formulations or antibody-drug conjugate formulations as described herein, refers to a formulation in which the antibody or antibody-drug conjugate therein essentially retains its physical and chemical identity and integrity upon storage. Various analytical techniques for measuring protein stability are available in the art (see, e.g., Peptide and Protein Drug Delivery, 247-301 (Vincent Lee Ed., Marcel Dekker, Inc., New York, N.Y., Pubs. 1991) and Jones, Adv. Drug Delivery Rev. 10:29-90, 1993). Exemplary techniques for measuring protein stability are also described herein (see Examples, infra). Stability can be measured at a selected temperature for a selected time period. For rapid testing, the formulation may be kept at a higher or "accelerated" temperature, for example, 40°C for 1 week to 1 month or more at which time stability is measured. In exemplary embodiments, the formulation is refractory to the formation of by-products of the component antibody protein, for example, high molecular weight aggregation products, low molecular weight degradation or fragmentation products, acidic species, chemical degradants, or mixtures thereof. The term "stability" refers to the length of time over which a molecular species such as an antibody retains its original chemical identity, for example, primary, secondary, and/or tertiary structure.

The phrase "pharmaceutically acceptable salt," as used herein, refers to pharmaceutically acceptable organic or inorganic salts of a compound. The compound can contain at least one amino group, and accordingly acid addition salts can be formed with the amino group. Exemplary salts include, but are not limited to, sulfate, trifluoroacetate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucuronate, saccharate, formate, benzoate, glutamate, methanesulfonate, ethanesulfonate, benzenesulfonate, p toluenesulfonate, and pamoate (i.e., 1,1' methylene bis -(2 hydroxy 3 naphthoate)) salts. A pharmaceutically acceptable salt may involve the inclusion of another molecule such as an acetate ion, a succinate ion or other counterion. The counterion may be any organic or inorganic moiety that stabilizes the charge on the parent compound. Furthermore, a pharmaceutically acceptable salt may have more than one charged atom in its structure. Instances where multiple charged atoms are part of the pharmaceutically acceptable salt can have multiple counter ions. Hence, a pharmaceutically acceptable salt can have one or more charged atoms and/or one or more counterion.

A "polypeptide" or "polypeptide chain" is a polymer of amino acid residues joined by peptide bonds, whether produced naturally or synthetically. Polypeptides of less than about 10 amino acid residues are commonly referred to as "peptides."

A "protein" is a macromolecule comprising one or more polypeptide chains. A protein may also comprise non-peptidic components, such as carbohydrate groups. Carbohydrates and other non-peptidic substituents may be added to a protein by the cell in which the protein is produced, and will vary with the type of cell. Proteins are defined herein in terms of their amino acid backbone structures; substituents such as carbohydrate groups are generally not specified, but may be present nonetheless.

The terms "amino-terminal" and "carboxyl-terminal" are used herein to denote positions within polypeptides. Where the context allows, these terms are used with reference to a particular sequence or portion of a polypeptide to denote proximity or relative position. For example, a certain sequence positioned carboxyl-terminal to a reference sequence within a polypeptide is located proximal to the carboxyl terminus of the reference sequence, but is not necessarily at the carboxyl terminus of the complete polypeptide.

The term "antibody" is used herein to denote immunoglobulin proteins produced by the body in response to the presence of an antigen and that bind to the antigen, as well as antigen-binding fragments and engineered variants thereof. Hence, the term "antibody" includes, for example, intact monoclonal antibodies comprising full-lengh immunoglobulin heavy and light chains (e.g., antibodies produced using hybridoma technology) and antigen-binding antibody fragments, such as F(ab')2 and Fab fragments. Genetically engineered intact antibodies and fragments, such as chimeric antibodies, humanized antibodies, single-chain Fv fragments, single-chain antibodies, diabodies, minibodies, linear antibodies, multivalent or multispecific (e.g., bispecific) hybrid antibodies, and the like are also included. Thus, the term "antibody" is used expansively to include any protein that comprises an antigen-binding site of an antibody and is capable of specifically binding to its antigen.

The term "genetically engineered antibodies" means antibodies wherein the amino acid sequence has been varied from that of a native antibody. Because of the relevance of recombinant DNA techniques in the generation of antibodies, one need not be confined to the sequences of amino acids found in natural antibodies; antibodies can be redesigned to obtain desired characteristics. The possible variations are many and range from the changing of just one or a few amino acids to the complete redesign of, for example, the variable or constant region. Changes in the constant region will, in general, be made in order to improve or alter characteristics such as, e.g., complement fixation, interaction with cells, and other effector functions. Typically, changes in the variable region will be made in order to improve the antigen-binding characteristics, improve variable region stability, or reduce the risk of immunogenicity.

An "antigen-binding site of an antibody" is that portion of an antibody that is sufficient to bind to its antigen. The minimum such region is typically a variable domain or a genetically engineered variant thereof. Single-domain binding sites can be generated from camelid antibodies (see Muyldermans and Lauwereys, J. Mol. Recog. 12:131-140, 1999; Nguyen et al., EMBO J. 19:921-930, 2000) or from VH domains of other species to produce single-domain antibodies ("dAbs"; see Ward et al., Nature 341:544-546, 1989; US Patent No. 6,248,516 to Winter et al.). In certain variations, an antigen-binding site is a polypeptide region having only 2 complementarity determining regions (CDRs) of a naturally or non-naturally (e.g., mutagenized) occurring heavy chain variable domain or light chain variable domain, or combination thereof (see, e.g., Pessi et al., Nature 362:367-369, 1993; Qiu et al., Nature Biotechnol. 25:921-929, 2007). More commonly, an antigen-binding site of an antibody comprises both a heavy chain variable (VH) domain and a light chain variable (VL) domain that bind to a common epitope. Within the context of the present invention, an antibody may include one or more components in addition to an antigen-binding site, such as, for example, a second antigen-binding site of an antibody (which may bind to the same or a different epitope or to the same or a different antigen), a peptide linker, an immunoglobulin constant region, an immunoglobulin hinge, an amphipathic helix (see Pack and Pluckthun, Biochem. 31:1579-1584, 1992), a non-peptide linker, an oligonucleotide (see Chaudri et al., FEBS Letters 450:23-26, 1999), a cytostatic or cytotoxic drug, and the like, and may be a monomeric or multimeric protein. Examples of molecules comprising an antigen-binding site of an antibody are known in the art and include, for example, Fv, single-chain Fv (scFv), Fab, Fab', F(ab')2, F(ab)c, diabodies, dAbs, minibodies, nanobodies, Fab-scFv fusions, bispecific (scFv)4-IgG, and bispecific (scFv)2-Fab. (See, e.g., Hu et al., Cancer Res. 56:3055-3061, 1996; Atwell et al., Molecular Immunology 33:1301-1312, 1996; Carter and Merchant, Curr. Opin. Biotechnol. 8:449-454, 1997; Zuo et al., Protein Engineering 13:361-367,2000; and Lu et al., J. Immunol. Methods 267:213-226, 2002.)

As used herein, the term "immunoglobulin" refers to a protein consisting of one or more polypeptides substantially encoded by immunoglobulin gene(s). One form of immunoglobulin constitutes the basic structural unit of native (i.e., natural) antibodies in vertebrates. This form is a tetramer and consists of two identical pairs of immunoglobulin chains, each pair having one light chain and one heavy chain. In each pair, the light and heavy chain variable regions (VL and VH) are together primarily responsible for binding to an antigen, and the constant regions are primarily responsible for the antibody effector functions. Five classes of immunoglobulin protein (IgG, IgA, IgM, IgD, and IgE) have been identified in higher vertebrates. IgG comprises the major class; it normally exists as the second most abundant protein found in plasma. In humans, IgG consists of four subclasses, designated IgG1, IgG2, IgG3, and IgG4. The heavy chain constant regions of the IgG class are identified with the Greek symbol γ. For example, immunoglobulins of the IgG1 subclass contain a γ1 heavy chain constant region. Each immunoglobulin heavy chain possesses a constant region that consists of constant region protein domains (CH1, hinge, CH2, and CH3; IgG3 also contains a CH4 domain) that are essentially invariant for a given subclass in a species. DNA sequences encoding human and non-human immunoglobulin chains are known in the art. (*See, e.g.,* Ellison et al., DNA 1:11-18, 1981; Ellison et al., Nucleic Acids Res. 10:4071-4079, 1982; Kenten et al., Proc. Natl. Acad. Sci. USA 79:6661-6665, 1982; Seno et al., Nuc. Acids Res. 11:719-726, 1983; Riechmann et al., Nature 332:323-327, 1988; Amster et al., Nuc. Acids Res. 8:2055-2065, 1980; Rusconi and Kohler, Nature 314:330-334, 1985; Boss et al., Nuc. Acids Res. 12:3791-3806, 1984; Bothwell et al., Nature 298:380-382, 1982; van der Loo et al., Immunogenetics 42:333-341, 1995; Karlin et al., J. Mol. Evol. 22:195-208, 1985; Kindsvogel et al., DNA 1:335-343, 1982; Breiner et al., Gene 18:165-174,1982; Kondo et al., Eur. J. Immunol. 23:245-249, 1993; and GenBank Accession No. J00228.) For a review of immunoglobulin structure and function, see Putnam, The Plasma Proteins, Vol V, Academic Press, Inc., 49-140, 1987; and Padlan, Mol. Immunol. 31:169-217, 1994. The term "immunoglobulin" is used herein for its common meaning, denoting an intact antibody, its component chains, or fragments of chains, depending on the context.

Full-length immunoglobulin "light chains" (about 25 Kd or 214 amino acids) are encoded by a variable region gene at the amino-terminus (encoding about 110 amino acids) and a by a kappa or lambda constant region gene at the carboxyl-terminus. Full-length immunoglobulin "heavy chains" (about 50 Kd or 446 amino acids) are encoded by a variable region gene (encoding about 116 amino acids) and a gamma, mu, alpha, delta, or epsilon constant region gene (encoding about 330 amino acids), the latter defining the antibody's isotype as IgG, IgM, IgA, IgD, or IgE, respectively. Within light and heavy chains, the variable and constant regions are joined by a "J" region of about 12 or more amino acids, with the heavy chain also including a "D" region of about 10 more amino acids. (*See generally* Fundamental Immunology (Paul, ed., Raven Press, N.Y., 2nd ed. 1989), Ch. 7).

An immunoglobulin light or heavy chain variable region (also referred to herein as a "light chain variable domain" ("VL domain") or "heavy chain variable domain" ("VH domain"), respectively) consists of a "framework" region interrupted by three hypervariable regions, also called "complementarity determining regions" or "CDRs." The framework regions serve to align the CDRs for specific binding to an epitope of an antigen. Thus, the term "hypervariable region" or "CDR" refers to the amino acid residues of an antibody that are primarily responsible for antigen binding. From amino-terminus to carboxyl-terminus, both VL and VH domains comprise the following framework (FR) and CDR regions: FR1, CDR1, FR2, CDR2, FR3, CDR3, FR4. The assignment of amino acids to each domain is in accordance with the definitions of Kabat, Sequences of Proteins of Immunological Interest (National Institutes of Health, Bethesda, MD, 1987 and 1991), or Chothia & Lesk, J. Mol. Biol. 196:901-917, 1987; Chothia et al., Nature 342:878-883, 1989. Kabat also provides a widely used numbering convention (Kabat numbering) in which corresponding residues between different heavy chains or between different light chains are assigned the same number. CDRs 1, 2, and 3 of a VL domain are also referred to herein, respectively, as CDR-L1, CDR-L2, and CDR-L3; CDRs 1, 2, and 3 of a VH domain are also referred to herein, respectively, as CDR-H1, CDR-H2, and CDR-H3.

Unless the context dictates otherwise, the term "monoclonal antibody" as used herein is not limited to antibodies produced through hybridoma technology. The term "monoclonal antibody" refers to an antibody that is derived from a single clone, including any eukaryotic, prokaryotic, or phage clone, and not the method by which it is produced.

The term "chimeric antibody" refers to an antibody having variable domains derived from a first species and constant regions derived from a second species. Chimeric immunoglobulins or antibodies can be constructed, for example by genetic engineering, from immunoglobulin gene segments belonging to different species. The term "humanized antibody," as defined infra, is not intended to encompass chimeric antibodies. Although humanized antibodies are chimeric in their construction (i.e., comprise regions from more than one species of protein), they include additional features (i.e., variable regions comprising donor CDR residues and acceptor framework residues) not found in chimeric immunoglobulins or antibodies, as defined herein.

The term "humanized VH domain" or "humanized VL domain" refers to an immunoglobulin VH or VL domain comprising some or all CDRs entirely or substantially from a non-human donor immunoglobulin (e.g., a mouse or rat) and variable region framework sequences entirely or substantially from human immunoglobulin sequences. The non-human immunoglobulin providing the CDRs is called the "donor" and the human immunoglobulin providing the framework is called the "acceptor." In some instances, humanized antibodies may retain non-human residues within the human variable domain framework regions to enhance proper binding characteristics (e.g., mutations in the frameworks may be required to preserve binding affinity when an antibody is humanized).

A "humanized antibody" is an antibody comprising one or both of a humanized VH domain and a humanized VL domain. Immunoglobulin constant region(s) need not be present, but if they are, they are entirely or substantially from human immunoglobulin constant regions.

A CDR in a humanized antibody is "substantially from" a corresponding CDR in a non-human antibody when at least 60%, at least 85%, at least 90%, at least 95% or 100% of corresponding residues (as defined by Kabat) are identical between the respective CDRs. In particular variations of a humanized VH or VL domain in which CDRs are substantially from a non-human immunoglobulin, the CDRs of the humanized VH or VL domain have no more than six (e.g., no more than five, no more than four, no more than three, no more than two, or nor more than one) amino acid substitutions across all three CDRs relative to the corresponding non-human VH or VL CDRs. The variable region framework sequences of an antibody VH or VL domain or, if present, a sequence of an immunoglobulin constant region, are "substantially from" a human VH or VL framework sequence or human constant region, respectively, when at least 85%, at least 90%, at least 95%, or 100% of corresponding residues defined by Kabat are identical. Hence, all parts of a humanized antibody, except possibly the CDRs, are entirely or substantially from corresponding parts of natural human immunoglobulin sequences.

Specific binding of an antibody to its target antigen means an affinity of at least 10⁶, 10⁷, 10⁸, 10⁹, or 10¹⁰ M⁻¹. Specific binding is detectably higher in magnitude and distinguishable from non-specific binding occurring to at least one unrelated target. Specific binding can be the result of formation of bonds between particular functional groups or particular spatial fit (e.g., lock and key type) whereas nonspecific binding is usually the result of van der Waals forces. Specific binding does not, however, necessarily imply that a monoclonal antibody binds one and only one target.

With regard to proteins as described herein, reference to amino acid residues corresponding to those specified by SEQ ID NO includes post-translational modifications of such residues.

The term "anti-CD 19 antibody" refers to an antibody that specifically binds to the human CD19 protein. In a preferred embodiement the anti-CD 19 antobidy comprises the CDRs of the light chain variable region of SEQ ID NO: 1 and the CDRs of the heavy chain variable region of SEQ ID NO:2. In another preferred embodiment, the anti-CD19 antobidy comprises the light chain variable region of SEQ ID NO: 1 and the heavy chain variable region of SEQ ID NO:2. In other preferred embodiments the anti-CD19 antibody includes a human constant region and is an IgG1 antibody.

The term "by-product" includes undesired products, which detract or diminish the proportion of therapeutic antibody-drug conjugate in a given formulation. Typical by-products include aggregates of the antibody-drug conjugate, fragments of the antibody-drug conjugate (for example, produced by degradation of the antibody protein by deamidation or hydrolysis or chemical degradation and fragmentation of the drug-linker), acidic variants of the antibody-drug conjugate, or mixtures thereof.

An antibody-drug conjugate (ADC) is an antibody conjugated to a cytotoxic drug typically via a linker. The linker may comprise a cleavable unit or may be non-cleavable. Cleavable units include, for example, disulfide containing linkers that are cleavable through disulfide exchange, acid-labile linkers that are cleavable at acidic pH, and linkers that are cleavable by hydrolases, esterases, peptidases, and glucoronidases (e.g., peptide linkers and glucoronide linkers). Non-cleavable linkers are believed to release drug via a proteolytic antibody degradation mechanism.

The term "high molecular weight aggregates" includes aggregates of the antibody-drug conjugate (ADC), as well as aggregates comprising fragments of the ADC (for example, produced by degradation of the polypeptide by, for example, hydrolysis) and aggregates comprising a mixtures of the ADC and such fragments. The presence of high molecular weight aggregates may be determined by, e.g., size-exclusion chromatography (SEC). Typically, high molecular weight aggregates are complexes which have a molecular weight which is greater than the therapeutic monomer ADC. In the case of an ADC in which the antibody component is a tetramer consisting of two identical pairs of immunoglobulin chains, each pair having one light chain and one heavy chain (e.g., of the IgG isotype), such aggregates are greater than about 150 kD. In the case, however, of an ADC in which the antibody component has a molecular weight greater than or less than that of a typical monospecific, tetrameric antibody protein consisting of two immunoglobulin light chains and two immunoglobulin heavy chains (e.g., single-chain antibodies or bispecific antibodies), the size of such aggregates can vary accordingly.

The term "low molecular weight degradation product" includes, for example, fragments of the antibody-drug conjugate (ADC) such as, for example, fragments brought about by deamidation or hydrolysis. The presence of low molecular weight degradation products may be determined by, e.g., size-exclusion chromatography (SEC). Typically, low molecular weight degradation products have a molecular weight that is less than the therapeutic monomer ADC. In the case of an ADC in which the antibody component is a tetramer consisting of two identical pairs of immunoglobulin chains, each pair having one light chain and one heavy chain (e.g., of the IgG isotype), such degradation products are less than about 150 kD. In the case, however, of an ADC in which the antibody component has a molecular weight greater than or less than that of a typical monospecific, tetrameric antibody protein consisting of two immunoglobulin light chains and two immunoglobulin heavy chains (e.g., single-chain antibodies or bispecific antibodies), the size of such degradation products can vary accordingly.

An "acidic variant" of an antibody-drug conjugate (ADC) of interest is an ADC variant that is more acidic than the experimental PI of the ADC. The presence of acid variants may be determined by, e.g., cation exchange chromatography or imaging capillary IEF (icIEF). An example of an acidic variant is a deamidated variant. Deamidated variants of a protein molecule are those in which one or more neutral amide side chain(s) have been converted to a residue with an overall acidic character (e.g., one or more asparagine residue(s) of the original polypeptide have been converted to aspartate).

The term "diluent" as used herein refers to a solution suitable for altering or achieving an exemplary or appropriate concentration or concentrations as described herein.

The term "container" refers to something into which an object or liquid can be placed or contained, e.g., for storage (for example, a holder, receptacle, vessel, or the like).

The term "administration route" includes art-recognized administration routes for delivering a therapeutic protein such as, for example, parenterally, intravenously, intramuscularly, or subcutaneously. For administration of an ADC for the treatment of cancer, administration into the systemic circulation by intravenous or subcutaneous administration may be desired. For treatment of a cancer characterized by a solid tumor, administration can be localized directly into the tumor, if so desired.

The term "treatment" refers to the administration of a therapeutic agent to a patient, who has a disease with the purpose to cure, heal, alleviate, delay, relieve, alter, remedy, ameliorate, improve or affect the disease.

The term "patient" includes human and other mammalian subjects that receive either prophylactic or therapeutic treatment.

The term "effective amount," "effective dose," or "effective dosage" refers to an amount that is sufficient to achieve or at least partially achieve the desired effect, e.g., sufficient to inhibit the occurrence or ameliorate one or more symptoms of a disease or disorder. An effective amount of a pharmaceutical composition is administered in an "effective regime." The term "effective regime" refers to a combination of amount of the composition being administered and dosage frequency adequate to accomplish prophylactic or therapeutic treatment of the disease or disorder.

The term "dosage unit form" (or "unit dosage form") as used herein refers to a physically discrete unit suitable as unitary dosages for a patient to be treated, each unit containing a predetermined quantity of active compound (an ADC in accordance with the present invention) calculated to produce the desired therapeutic effect in association with the required pharmaceutical carrier, diluent, or excipient. The specification for the dosage unit forms of the invention are dictated by and directly dependent on the unique characteristics of the active compound and the particular therapeutic effect to be achieved, and the limitations inherent in the art of compounding such an active compound for the treatment of patients.

Actual dosage levels of an ADC in a formulation of the present invention may be varied so as to obtain an amount of the ADC that is effective to achieve a desired therapeutic response for a particular patient, composition, and mode of administration, without being toxic to the patient. The selected dosage level will depend upon a variety of pharmacokinetic factors including the activity of the particular compositions of the present invention employed, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well-known in the medical arts.

A "cytotoxic effect" refers to the depletion, elimination and/or the killing of a target cell. A "cytotoxic agent" refers to an agent that has a cytotoxic effect on a cell.

A "cytostatic effect" refers to the inhibition of cell proliferation. A "cytostatic agent" refers to an agent that has a cytostatic effect on a cell, thereby inhibiting the growth and/or expansion of a specific subset of cells.

Two amino acid sequences have "100% amino acid sequence identity" if the amino acid residues of the two amino acid sequences are the same when aligned for maximal correspondence. Sequence comparisons can be performed using standard software programs such as those included in the LASERGENE bioinformatics computing suite, which is produced by DNASTAR (Madison, Wisconsin). Other methods for comparing two nucleotide or amino acid sequences by determining optimal alignment are well-known to those of skill in the art. (*See, e.g.,* Peruski and Peruski, The Internet and the New Biology: Tools for Genomic and Molecular Research (ASM Press, Inc. 1997); Wu et al. (eds.), "Information Superhighway and Computer Databases of Nucleic Acids and Proteins," in Methods in Gene Biotechnology 123-151 (CRC Press, Inc. 1997); Bishop (ed.), Guide to Human Genome Computing (2nd ed., Academic Press, Inc. 1998).) Two amino acid sequences are considered to have "substantial sequence identity" if the two sequences have at least 80%, at least 85%, at least 90%, or at least 95% sequence identity relative to each other.

Percentage sequence identities are determined with antibody sequences maximally aligned by the Kabat numbering convention. After alignment, if a subject antibody region (e.g., the entire variable domain of a heavy or light chain) is being compared with the same region of a reference antibody, the percentage sequence identity between the subject and reference antibody regions is the number of positions occupied by the same amino acid in both the subject and reference antibody region divided by the total number of aligned positions of the two regions, with gaps not counted, multiplied by 100 to convert to percentage.

The term "pharmaceutical formulation" refers to a preparation which is in such form as to permit the biological activity of the active ingredient to be effective (when administered to a subject), and which contains no additional components which are unacceptably toxic to a subject to which the formulation would be administered. Such formulations are sterile.

Compositions or methods "comprising" one or more recited elements may include other elements not specifically recited.

Reference to a numerical range herein (e.g., "X to Y" or "from X to Y") includes the endpoints defining the range and all values falling within the range.

As used herein, the term "about" denotes an approximate range of plus or minus 10% from a specified value. For instance, the language "about 20%" encompasses a range of 18-22%. As used herein, about also includes the exact amount. Hence "about 20%" means "about 20%" and also "20%."

### BRIEF DESCRIPTION OF THE DRAWINGS

Figure 1A provides an assessment of the stability of the humanized anti-CD19 antibody, hBU12, in various buffers at various pH values.
Figure 1B provides an assessment of the stability of the humanized anti-CD 19 antibody hBU12 conjugated to MMAF, SGN-CD19A, in various buffers at various pH values.
Figure 2 provides an assessment of the effect of pH on the chemical stability of the humanized anti-CD 19 antibody hBU12 conjugated to MMAF, SGN-CD19A.
Figure 3A demonstrates the effect of freeze/thaw on subvisible particles in hBU12 formulation without polysorbate 80 (0 and 1 freeze/thaw cycle).
Figure 3B demonstrates the effect of freeze/thaw on subvisible particles in hBU12 formulation without polysorbate 80 (2 and 3 freeze/thaw cycles).
Figure 4 demonstrates the effect of freeze/thaw on subvisible particles in hBU12 formulation with 0.01% polysorbate 80 (0, 1, 2 and 3 freeze/thaw cycles).
Figure 5 demonstrates the effect of freeze/thaw on subvisible particles in hBU12 formulation with 0.02% polysorbate 80 (0, 1, 2 and 3 freeze/thaw cycles).
Figure 6 demonstrates the effect of freeze/thaw on subvisible particles in hBU12 formulation with 0.03% polysorbate 80 (0, 1, 2 and 3 freeze/thaw cycles).
Figure 7 summarizes data on the effect of two sugars, sucrose and trehalose, on the stability of CD19 ADC lyophilized formulations.
Figure 8 provides the structure of SGN-CD19A, a CD19 antibody drug conjugate (ADC) comprising the mcMMAF drug linker and the humanized antibody hBU12.

### DETAILED DESCRIPTION

This invention defined in the claims,provides stable formulations of anti-CD 19 antibodies and of ADC's comprising anti-CD 19 antibodies. The formulations include a phosphate buffer, a sugar, and polysorbate-80. The formulations provide stability for the anti-CD19 antibodies and ADC's comprising anti-CD 19 antibodies in both liquid and lyophilized embodiments.

The invention provides a stable formulation of a humanized anti-CD19 antibody, hBU12, as described below. While working with the hBU12 antibody, it was discovered that the hBU12 antibody was unexpectedly unstable and oxidized in the presence of light. An oxidized impurity was detected after incubation of the antibody in the presence of light. Many formulations were tested for their ability to improve the stability of the antibody in the presence of light and to decrease the amount the oxidized impurity. However, the greatest decrease in the oxidized impurity came with use of phosphate buffer in the formulation. The decrease in oxidation associated with light was seen in formulations of unconjugated hBU12 antibody, as well as formulations of the hBU12 antibody conjugated to a cytotoxic agent, for example, mcMMAF.

Also disclosed are other aspects of the hBU12 formulation that improve the stability of the antibody or antibody drug conjugate. The optimized aspects include, for example, buffer pH, addition of sugars, and addition of surfactants.

### Anti-CD19 antibodies, ADC's, and formulations

The pharmaceutical formulations of the present invention comprise a humanized antibody, or an antibody-drug conjugate, that binds specifically to the human CD19 protein. As used herein, the term "CD19" means a human CD19 protein or cluster of differentiation 19 protein. The amino acid sequence of human CD19 is known and is disclosed, e.g., at NCBI Reference Sequence: NP_001171569.1. The CD19 protein is a marker for B-cell deveopment and is expressed on B cells at many stages of B cell development.

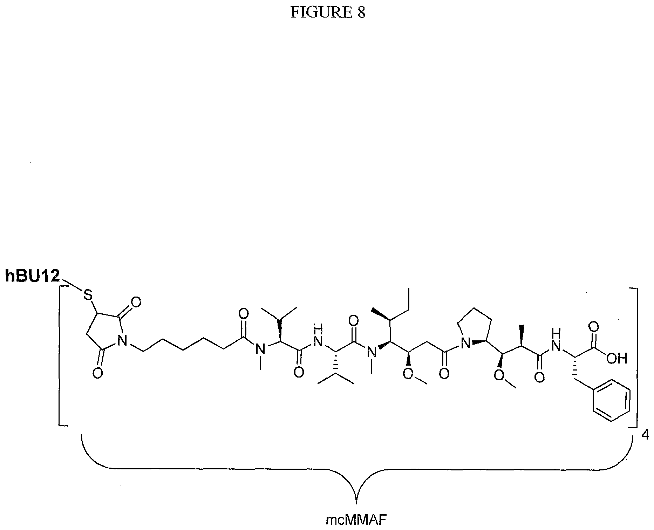

The formulations disclosed herein are stable formulations of the SGN-19A therapeutic agent. SGN-CD19A is produced by the conjugation of drug-linker intermediate maleimidocaproyl monomethyl auristatin F (mcMMAF) to the humanized antibody hBU12 (Figure 8). The points of attachment are cysteines produced by reduction of inter-chain disulfides. SGN-CD19A has an average of four drugs per antibody molecule.

Methods of making the hBU12 antibody are disclosed, e.g., at US Patent No. 7,968,687. The amino acid sequence of the light chain variable region of hBU12 is provided herein as SEQ ID NO:1. The amino acid sequence of the heavy chain variable region of hBU12 is provided herein as SEQ ID NO:2. hBU12 is an IgG1 antibody and the variable regions are joined to human heavy and light constant regions. US Patent No. 7,968,687 also provides methods for the synthesis of mcMMAF and its conjugation to hBU12.

SGN-CD19A, therefore, is an antibody-drug conjugate (ADC) that delivers mcMMAF to CD19-positive cells. mcMMAF is a tubulin-binding molecule. SGN-CD19A has a proposed multi-step mechanism of action initiated by binding to their target on the cell surface and subsequent internalization. After cell surface binding, internalization, and trafficking of SGN-CD19A through the endocytic pathway, proteolytic degradation of hBU12 in the lysosomes releases the cysteine adduct of the drug linker in the form of cys-mcMMAF, which then becomes available for tubulin binding. *See, e.g.,* Doronina et al., Nat Biotechnol 21:778-84 (2003) and Doronina et al., Bioconjug Chem 17: 114-24 (2006). cys-mcMMAF and mcMMAF are used interchangeably herein. Binding of the released drug to tubulin disrupts the cellular microtubule network, leading to G2/M phase cell cycle arrest and subsequent onset of apoptosis in the targeted cell.

The antibody component of SGN-CD19A can degrade in the presence of light. The formulations disclosed herein provide improved stability of SGN-CD19A. Moreover, the formulations disclosed herein allow choice of liquid or lyophilized formulations, depending on the needs of the user.

### Formulations and Excipients in General

Excipients are additives that either impart or enhance the stability and delivery of a drug product (e.g., an antibody or ADC). Regardless of the reason for their inclusion, excipients are an integral component of a formulation and therefore need to be safe and well tolerated by patients. For protein drugs, the choice of excipients is particularly important because they can affect both efficacy and immunogenicity of the drug. Hence, protein formulations need to be developed with appropriate selection of excipients that afford suitable stability, safety, and marketability.

The principal challenge in developing formulations for proteins is stabilizing the product against the stresses of manufacturing, shipping and storage. The role of formulation excipients is to provide stabilization against these stresses. Excipients are also employed to reduce viscosity of high concentration protein formulations in order to enable their delivery and enhance patient convenience. In general, excipients can be classified on the basis of the mechanisms by which they stabilize proteins against various chemical and physical stresses. Some excipients are used to alleviate the effects of a specific stress or to regulate a particular susceptibility of a specific protein. Other excipients have more general effects on the physical and covalent stabilities of proteins. The excipients described herein are organized either by their chemical type or their functional role in formulations. Brief descriptions of the modes of stabilization are provided when discussing each excipient type.

Given the teachings and guidance provided herein, those skilled in the art will know what amount or range of excipient can be included in any particular formulation to achieve a biopharmaceutical formulation of the invention that promotes retention in stability of the antibody or ADC. For example, the amount and type of a salt to be included in a biopharmaceutical formulation of the invention is selected based on the desired osmolality (i.e., isotonic, hypotonic or hypertonic) of the final solution as well as the amounts and osmolality of other components to be included in the formulation.

Further, where a particular excipient is reported in molar concentration, those skilled in the art will recognize that the equivalent percent (%) w/v (e.g., (grams of substance in a solution sample/mL of solution) X 100%) of solution is also contemplated.

The stability of a pharmacologically active protein formulation is usually observed to be maximal in a narrow pH range. This pH range of optimal stability needs to be identified early during pre-formulation studies. Several approaches, such as accelerated stability studies and calorimetric screening studies, are useful in this endeavor. See, e.g., Remmele R. L. Jr., et al., Biochemistry, 38(16): 5241-7 (1999). Once a formulation is finalized, the protein must be manufactured and maintained throughout its shelf-life. Hence, buffering agents are almost always employed to control pH in the formulation. As disclosed above, the hBU12 antibody exhibited improved photostability in the presence of phosphate buffers, as compared to other buffering agents. The optimal pH value for stability was investigated and found to range between pH values of 5.5 and 6.5. In preferred embodiments the pH value is about 6.0. In further embodiments, the pH value is 6.0.

The phosphate buffering compound may be present in any amount suitable to maintain the pH of the formulation at a predetermined level. In one embodiment, the pH buffering concentration is between 0.1 mM and 500 mM (1 M). For example, it is contemplated that the phosphate buffering agent is at least 0.1, 0.5, 0.7, 0.8 0.9, 1.0, 1.2, 1.5, 1.7, 2, 3, 4, 5, 6, 7, 8, 9, 10, 11, 12, 13, 14, 15, 16, 17, 18, 19, 20, 25, 30, 40, 50, 60, 70, 80, 90, 100, 200, or 500 mM.

In one aspect of the present pharmaceutical formulations, a stabilizer (or a combination of stabilizers) is added to prevent or reduce storage-induced aggregation and chemical degradation. A hazy or turbid solution upon reconstitution indicates that the protein has precipitated or at least aggregated. The term "stabilizer" means an excipient capable of preventing aggregation or physical degradation, including chemical degradation (for example, autolysis, deamidation, oxidation, etc.) in an aqueous state. Stabilizers contemplated include, but are not limited to, sucrose, trehalose, mannose, maltose, lactose, glucose, raffinose, cellobiose, gentiobiose, isomaltose, arabinose, glucosamine, fructose, mannitol, sorbitol, glycine, arginine HCL, poly-hydroxy compounds, including polysaccharides such as dextran, starch, hydroxyethyl starch, cyclodextrins, N-methyl pyrollidene, cellulose and hyaluronic acid, sodium chloride. See, e.g., Carpenter et al., Develop. Biol. Standard 74:225, (1991). In the present formulations, the stabilizer is incorporated in a concentration of about 0.1, 0.5, 0.7, 0.8 0.9, 1.0, 1.2, 1.5, 1.7, 2, 3, 4, 5, 6, 7, 8, 9, 10, 11, 12, 13, 14, 15, 16, 17, 18, 19, 20, 30, 40, 50, 60, 70, 80, 90, 100, or 200 mg/ml. In certain embodiments of the present invention, sucrose or trehalose are used as stabilizing agents.

The formulations also include appropriate amounts of bulking and osmolarity regulating agents. Bulking agents include, for example and without limitation, mannitol, glycine, sucrose, polymers such as dextran, polyvinylpyrolidone, carboxymethylcellulose, lactose, sorbitol, trehalose, or xylitol. In the invention, the bulking agent is sucrose. The bulking agent is incorporated in a concentration of about 0.1, 0.5, 0.7, 0.8 0.9, 1.0, 1.2, 1.5, 1.7, 2, 3, 4, 5, 6, 7, 8, 9, 10, 11, 12, 13, 14, 15, 16, 17, 18, 19, 20, 30, 40, 50, 60, 70, 80, 90, 100, or 200 mg/ml..

Surfactants are commonly used in protein formulations to prevent surface-induced degradation. Surfactants are amphipathic molecules with the capability of out-competing proteins for interfacial positions. Hydrophobic portions of the surfactant molecules occupy interfacial positions (e.g., air/liquid), while hydrophilic portions of the molecules remain oriented towards the bulk solvent. At sufficient concentrations (typically around the detergent's critical micellar concentration), a surface layer of surfactant molecules serve to prevent protein molecules from adsorbing at the interface. Thereby, surface-induced degradation is minimized. Surfactants contemplated herein include, without limitation, fatty acid esters of sorbitan polyethoxylates, i.e. polysorbate 20 and polysorbate 80. The two differ only in the length of the aliphatic chain that imparts hydrophobic character to the molecules, C-12 and C-18, respectively. Accordingly, polysorbate-80 is more surface-active and has a lower critical micellar concentration than polysorbate-20.

In the disclosed formulations, the surfactant is incorporated in a concentration of about 0.01 to about 0.5 g/L. In formulations provided, the surfactant concentration is 0.005, 0.01, 0.02, 0.03, 0.05, 0.06, 0.07, 0.08, 0.09, 0.1, 0.2, 0.3, 0.4, 0.5, 0.6, 0.7, 0.8, 0.9 or 1.0 g/L (also referred to as % weight/volume). The surfactant of the invention is polysorbate-80.

### Exemplary formulations

The antibody and ADC formulations disclosed herein are suitable for both liquid and lyophilized formulations. That is, the antibody and ADC formulations, e.g., of SGN-CD19A, can be prepared in the disclosed concentrations and stored as a liquid formulation until administered to a patient. Alternatively, a liquid formulation can be prepared, e.g., of SGN-CD19A, in the disclosed concentrations then lyophilized and stored in state, until reconstituted and administered to a patient.

The disclosed stable formulation comprises an anti-CD 19 antibody at a concentration of between 5 and 25 mg/ml. Preferably, the anti-CD 19 antibody is included at between 10 and 20 mg/ml. Preferably, the anti-CD19 antibody is included at a concentration between 12.5 and 17.5 mg/ml. Preferably, the anti-CD19 antibody is included at a concentration between 14 and 16 mg/ml. Preferably, the anti-CD19 antibody is included at a concentration of about 15 mg/ml. Preferably, the anti-CD 19 antibody is included at a concentration of 15 mg/ml.

The disclosed stable formulation comprises an anti-CD19 antibody conjugated to a cytotoxic agent at a concentration of between 5 and 25 mg/ml. Preferably, the anti-CD19 antibody conjugated to a cytotoxic agent is included at between 10 and 20 mg/ml. Preferably, the anti-CD 19 antibody conjugated to a cytotoxic agent is included at a concentration between 12.5 and 17.5 mg/ml. Preferably, the anti-CD19 antibody conjugated to a cytotoxic agent is included at a concentration between 14 and 16 mg/ml. Preferably, the anti-CD19 antibody conjugated to a cytotoxic agent is included at a concentration of about 15 mg/ml. Preferably, the anti-CD 19 antibody conjugated to a cytotoxic agent is included at a concentration of 15 mg/ml.

The disclosed stable formulation comprises an anti-CD 19 antibody conjugated to an auristatin at a concentration of between 5 and 25 mg/ml. Preferably, the anti-CD 19 antibody conjugated to an auristatin is included at between 10 and 20 mg/ml. Preferably, the anti-CD 19 antibody conjugated to an auristatin is included at a concentration between 12.5 and 17.5 mg/ml. Preferably, the anti-CD 19 antibody conjugated to an auristatin is included at a concentration between 14 and 16 mg/ml. Preferably, the anti-CD19 antibody conjugated to an auristatin is included at a concentration of about 15 mg/ml. Preferably, the anti-CD19 antibody conjugated to an auristatin is included at a concentration of 15 mg/ml.

The stable formulation comprises an anti-CD 19 antibody conjugated to MMAF at a concentration of between 5 and 25 mg/ml. Preferably, the anti-CD19 antibody conjugated to MMAF is included at between 10 and 20 mg/ml. Preferably, the anti-CD 19 antibody conjugated to MMAF is included at a concentration between 12.5 and 17.5 mg/ml. Preferably, the anti-CD19 antibody conjugated to MMAFis included at a concentration between 14 and 16 mg/ml. In the invention, the anti-CD19 antibody conjugated to MMAF is included at a concentration of about 15 mg/ml.

The stable formulation comprises an anti-CD19 antibody comprising a light chain variable region of SEQ ID NO:1 and a heavy chain variable region of SEQ ID NO:2 that is conjugated to MMAF at a concentration of between 5 and 25 mg/ml. Preferably, the anti-CD19 antibody comprising a light chain variable region of SEQ ID NO:1 and a heavy chain variable region of SEQ ID NO:2 that is conjugated to MMAF is included at between 10 and 20 mg/ml. Preferably, the anti-CD 19 antibody comprising a light chain variable region of SEQ ID NO: 1 and a heavy chain variable region of SEQ ID NO:2 that is conjugated to MMAF is included at a concentration between 12.5 and 17.5 mg/ml. Preferably, the anti-CD19 antibody comprising a light chain variable region of SEQ ID NO: 1 and a heavy chain variable region of SEQ ID NO:2 that is conjugated to MMAF is included at a concentration between 14 and 16 mg/ml. In the invention, the anti-CD 19 antibody comprising a light chain variable region of SEQ ID NO: 1 and a heavy chain variable region of SEQ ID NO:2 that is conjugated to MMAF is included at a concentration of about 15 mg/ml.

The stable formulation comprises a phosphate buffer with a pH value between 5.0 and 7.0. In other embodiments, the stable formulation comprises a phosphate buffer with a pH value between 5.5 and 6.5. In other embodiments, the stable formulation comprises a phosphate buffer with a pH value between 5.8 and 6.2. In other embodiments, the stable formulation comprises a phosphate buffer with a pH value between 5.9 and 6.1. In other embodiments, the stable formulation comprises a phosphate buffer with a pH value of about 6.0. In other embodiments, the stable formulation comprises a phosphate buffer with a pH value of 6.0

The stable formulation comprises between 0.0% and 1% (W/V) polysorbate 80. Preferably, the stable formulation comprises between 0.05% and 0.5% (W/V) polysorbate 80. Preferably, the stable formulation comprises between 0.1% and 0.3% (W/V) polysorbate 80. In the invention, the stable formulation comprises about 0.2% (W/V) polysorbate 80.

The stable formulation comprises between 20 and 100 mg/ml sucrose. Preferably, the stable formulation comprises between 30 and 90 mg/ml sucrose. Preferably, the stable formulation comprises between 40 and 80 mg/ml sucrose. Preferably, the stable formulation comprises between 50 and 70 mg/ml sucrose. Preferably, the stable formulation comprises between 55 and 65 mg/ml sucrose. In the invention, the stable formulation comprises about 60 mg/ml sucrose.

The disclosed stable formulation comprises an anti-CD 19 antibody at a concentration of about 15 mg/ml in 10 mM potassium phosphate, at a pH of about 6.0, with about 0.02% w/v polysorbate 80, and about 60 mg/ml sucrose.

The disclosed stable formulation comprises an anti-CD 19 antibody at a concentration of 15 mg/ml in 10 mM potassium phosphate, at pH 6.0, with 0.02% w/v polysorbate 80, and 60 mg/ml sucrose.

The disclosed stable formulation comprises an anti-CD 19 antibody conjugated to a cytotoxic agent at a concentration of about 15 mg/ml in 10 mM potassium phosphate, at a pH of about 6.0, with about 0.02% w/v polysorbate 80, and about 60 mg/ml sucrose.

The disclosed stable formulation comprises an anti-CD 19 antibody conjugated to a cytotoxic agent at a concentration of 15 mg/ml in 10 mM potassium phosphate, at pH 6.0, with 0.02% w/v polysorbate 80, and 60 mg/ml sucrose.

The disclosed stable formulation comprises an anti-CD 19 antibody conjugated to an auristatin at a concentration of about 15 mg/ml in 10 mM potassium phosphate, at a pH of about 6.0, with about 0.02% w/v polysorbate 80, and about 60 mg/ml sucrose.

The disclosed stable formulation comprises an anti-CD 19 antibody conjugated to an auristatin at a concentration of 15 mg/ml in 10 mM potassium phosphate, at pH 6.0, with 0.02% w/v polysorbate 80, and 60 mg/ml sucrose.

In the invention, the stable formulation comprises an anti-CD19 antibody conjugated to mcMMAF at a concentration of about 15 mg/ml in 10 mM potassium phosphate, at a pH of about 6.0, with about 0.02% w/v polysorbate 80, and about 60 mg/ml sucrose.

In the invention, the stable formulation comprises an anti-CD 19 antibody conjugated to mcMMAF at a concentration of 15 mg/ml in 10 mM potassium phosphate, at pH 6.0, with 0.02% w/v polysorbate 80, and 60 mg/ml sucrose.

In the invention, the stable formulation comprises comprises an anti-CD19 antibody comprising a light chain variable region of SEQ ID NO:1 and a heavy chain variable region of SEQ ID NO:2 that is conjugated to mcMMAF at a concentration of about 15 mg/ml in 10 mM potassium phosphate, at a pH of about 6.0, with about 0.02% w/v polysorbate 80, and about 60 mg/ml sucrose.

In the invention, the stable formulation comprises comprises an anti-CD19 antibody comprising a light chain variable region of SEQ ID NO:1 and a heavy chain variable region of SEQ ID NO:2 that is conjugated to mcMMAF at a concentration of 15 mg/ml in 10 mM potassium phosphate, at pH 6.0, with 0.02% w/v polysorbate 80, and 60 mg/ml sucrose.

### Methods of Preparation

The stable formulation of a CD19 antibody or ADC can be prepared as either a liquid or a lyophilized preparation. As disclosed herein, both the liquid and lyophilized versions of the formulation are stable over time. Once a liquid formulation is made it can then be stored before. Preferably storage of the liquid version will occur at or about 4°C.

For a lyophilized version of the stable formulation, lyophilization is carried out using techniques common in the art. See, e.g., Tang et al., Pharm Res. 21:191-200, (2004) and Chang et al., Pharm Res. 13:243-9 (1996). A lyophilization cycle is, in one aspect, composed of three steps: freezing, primary drying, and secondary drying. See, e.g., A. P. Mackenzie, Phil Trans R Soc London, Ser B, Biol 278:167 (1977). In the freezing step, the solution is cooled to initiate ice formation. Furthermore, this step induces the crystallization of the bulking agent. The ice sublimes in the primary drying stage, which is conducted by reducing chamber pressure below the vapor pressure of the ice, using a vacuum and introducing heat to promote sublimation. Finally, adsorbed or bound water is removed at the secondary drying stage under reduced chamber pressure and at an elevated shelf temperature. The process produces a material known as a lyophilized cake. Thereafter the cake can be reconstituted with either sterile water or suitable diluent for injection.

The standard reconstitution practice for lyophilized material is to add back a volume of pure water or sterile water for injection (WFI) (typically equivalent to the volume removed during lyophilization), although dilute solutions of antibacterial agents are sometimes used in the production of pharmaceuticals for parenteral administration. See, e.g, Chen, Drug Development and Industrial Pharmacy, 18:1311-1354 (1992). Accordingly, methods are provided for preparation of reconstituted stable formulations comprising the step of adding a diluent to a lyophilized CD19 ADC formulation.

The lyophilized material may be reconstituted as an aqueous solution. A variety of aqueous carriers, e.g., sterile water for injection, or water with appropriate amounts of surfactants (for example, an aqueous suspension that contains the active compound in admixture with excipients suitable for the manufacture of aqueous suspensions).

The stable CD19 ADC formulations disclosed herein are administered to patients in need of treatment, e.g., patients with cancer that express extracellular CD19, e.g., non-hodgkin lymphoma or acute lymphoblastic leukemia or patients with an autoimmune disease that responds to treatment with a CD19 ADC. Typically, the CD19 formulations, either liquid or reconstituted lyophilized formulations, are administered intravenously.

Single or multiple administrations of the compositions are carried out with the dose levels and pattern being selected by the treating physician. For the prevention or treatment of disease, the appropriate dosage depends on the type of disease to be treated, as defined above, the severity and course of the disease, whether drug is administered for preventive or therapeutic purposes, previous therapy, the patient's clinical history and response to the drug, and the discretion of the attending physician.

As an additional aspect, the disclosure includes kits which comprise one or more liquid or lyophilized compositions packaged in a manner which facilitates their use for administration to subjects. Such a kit may include a stable pharmaceutical formulation described herein (e.g., a composition comprising a CD19 ADC, such as SGN-CD19A), packaged in a container such as a sealed bottle or vessel, with a label affixed to the container or included in the package that describes use of the compound or composition in practicing the method. The pharmaceutical formulation may be packaged in the container such that the amount of headspace in the container (e.g., the amount of air between the liquid formulation and the top of the container) is very small. Preferably, the amount of headspace is negligible (i.e., almost none). The kit may contain a first container having a lyophilized stable CD19 ADC formulation, such as SGN-CD19A, and a second container having a physiologically acceptable reconstitution solution for the composition. In one aspect, the pharmaceutical formulation is packaged in a unit dosage form. The kit may further include a device suitable for administering the pharmaceutical formulation according to a specific route of administration. Preferably, the kit contains a label that describes use of the pharmaceutical formulations.

The following examples are not intended to be limiting but only exemplary of specific embodiments of the invention, which is defined by the claims.

### EXAMPLES

### Example 1: Optimization of antibody photostability

Antibody hBU12 was observed to be unstable in the presence of light. Formulation studies indicated that buffer composition could influence the extent of photo-oxidation in hBU12. The following buffers were tested under intense light for their effect on the photostability of hBU12: 10 mM Na phosphate pH 6.5; 10 mM citrate, pH 6.5; and 10 mM histidine, pH 6.5. Each formulation had an hBU12 concentration of ∼ 7 mg/mL. The samples were held in a photochamber and exposed to intense light over a period of twenty-four hours. Photo oxidation ofhBU12 resulted increase in the oxidized Fab. The data in Table 1 shows change in oxidized Fab for each formulation over time and light exposure. The data indicates that the sodium phosphate formulation showed superior stability compared to citrate and histidine buffers.

**Table 1: Effect of buffer on antibody photostability in intense light.**

| | **Percent Oxidized Fab** | | |
|---|---|---|---|
| **Intense Light Exposure D ura tion** | **Histidine** | **Sodium Phosphate** | **Citrate** |
| T=0hrs | 7.6% | 7.3% | 8.0% |
| T=6hrs | 11.3% | 9.2% | 18.9% |
| T=18hrs | 21.0% | 12.2% | 36.2% |
| T=24hrs | 23.6% | 13.2% | 43.8% |
| T=24hrs Dark Control | 7.4% | 7.0% | 7.8% |

The next study investigated the effect of both intense light and ambient light exposure on another set of formulations. The following buffers were tested for their effect on the photostability of hBU12: 10 mM histidine, pH 6.0; 10 mM potassium phosphate, pH 6.0; 10 mM acetate, pH 6.0; and 10 mM succinate, pH 6.0. The concentration of hBU12 was approximately 4 mg/mL in each formulation.

Results are shown in Tables 2 and 3. When tested in intense light, hBU12 antibody formulated in a phosphate buffer at pH 6.0 was more photostable than the same antibody formulated in histidine, acetate or succinate buffers. See, e.g., Table 2. Table 3 provides the results in ambient light. Again, hBU12 antibody formulated in a phosphate buffer at pH 6.0 was more photostable than the same antibody formulated in histidine, acetate or succinate buffers.

**Table 2: Effect of buffer on antibody photostability in intense light.**

| | **Percent Oxidized Fab** | | | |
|---|---|---|---|---|
| **Intense light exposure duration (days)** | **Histidine** | **K Phosphate** | **Acetate** | **Succinate** |
| T=0hrs | 6.6% | 5.1% | 6.5% | 6.9% |
| T=12hrs | 10.9% | 6.7% | 8.1% | 10.1% |
| T=24hrs | 14.9% | 7.1% | 11.0% | 12.5% |
| T=24hrs Dark Control | 6.5% | 4.2% | 6.5% | 7.1% |

**Table 3: Effect of buffer on antibody photostability in ambient light.**

| | **Percent Oxidized Fab** | | | |
|---|---|---|---|---|
| **Ambient light exposure duration (days)** | **Histidine** | **K Phosphate** | **Acetate** | **Succinate** |
| T=0d | 6.6% | 5.1% | 6.5% | 6.9% |
| T=3d | 7.9% | 4.0% | 6.7% | 7.9% |
| T=11d | 9.8% | 4.5% | 7.6% | 8.6% |
| T=11d Dark Control | 6.8% | 3.1% | 6.3% | 7.0% |

An additional photostablity study was conducted for two hBU12 antibody formulations. For this study, the photostability of hBU12 antibody formulated in 10 mM histidine, pH 6.0 was compared to 10 mM K phosphate, pH 6.0 at an hBU12 concentration of ∼ 6 mg/mL. The formulations were subjected to intense light and the oxidation results are summarized in Table 4.

The results shown in Table 4 indicate that hBU12 antibody formulated in a potassium phosphate buffer at pH 6.0 was more photostable than the same antibody formulated in histidine buffer.

**Table 4: Photostability of potassium phosphate and histidine formulations in intense light**

| **Intense light exposure duration (hours)** | **% Oxidized Fab** | |
|---|---|---|
| | **Histidine** | **K Phosphate** |
| 0 | 8.7% | 8.6% |
| 19 h | 18.3% | 12.5% |
| 19 h Dark Control | 9.0% | 8.6% |

The hBU12 antibody was conjugated to the auristatin monomethylauristatin F (MMAF) using a maleimidocproyl (mc) linker. The structure of the mc-MMAF drug linker, methods of making it, and methods of conjugating the drug linker to the hBU12 antibody are disclosed, e.g., in McDonagh et al., WO2009/052431.

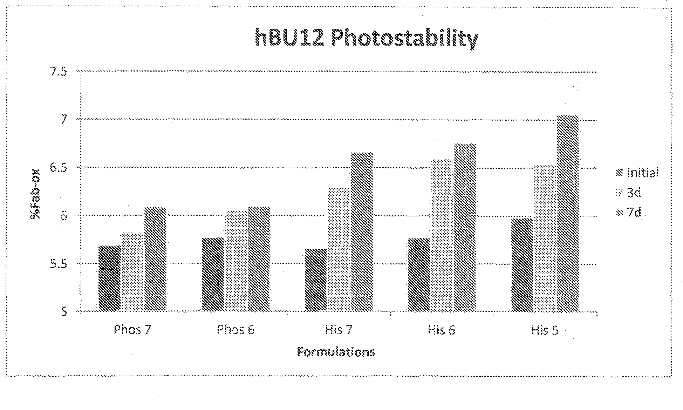

The effect of buffer type and pH on the photostability of the hBU12 mAb was investigated at ambient light exposure. Formulations were prepared in 10mM histidine buffers at pH 5.0 (H5), pH 6.0 (H6), and pH 7.0 (H7), as well as 10mM potassium phosphate buffers at pH 6.0 (P6) and pH 7.0 (P7). The photostability of the hBU12 antibody was measured at zero, three and seven days of ambient light exposure. The monoclonal antibody results are shown in figure 1A. As expected, the monoclonal antibody was most photostable in phosphate buffer at pH 6.0 (P6).

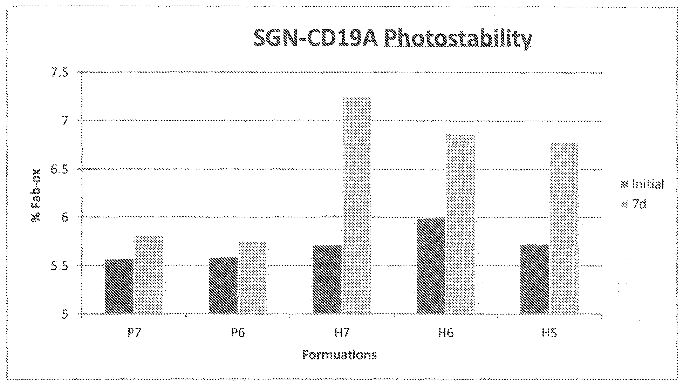

Phosphate buffer also stabilized the hBU12 ADC in the presence of light. The evaluation was done with phosphate and histidine buffers, at pH values ranging from 5.0 to 7.0. Formulations were prepared in 10mM histidine buffers at pH 5.0 (H5), pH 6.0 (H6), and pH 7.0 (H7), and 10mM potassium phosphate buffers at pH 6.0 (P6) and pH 7.0 (P7) The photostability of the CD19-ADC comprising hBU12 and MMAF was measured at zero and seven days of ambient light exposure. As before, the photostability was assessed by measuring the amount of oxidized Fab or ADC produced under various conditions Figure 1B shows that the CD19 ADC comprising hBU12 and MMAF was most photostable in phosphate buffer at pH 6.0 (P6).

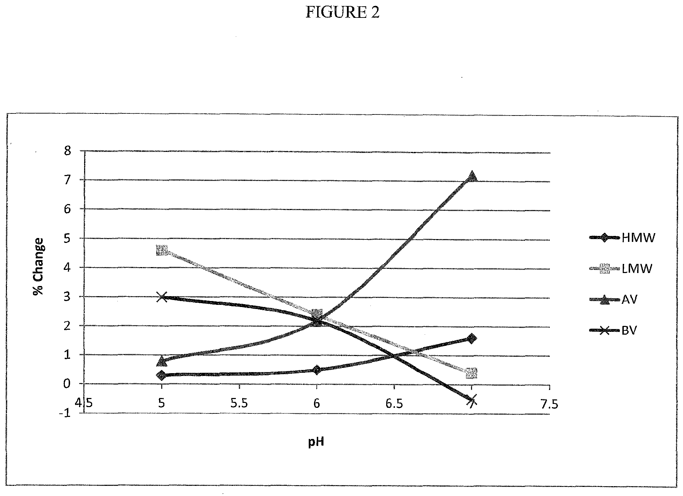

The effect of pH on the chemical stability of a CD19 ADC comprising hBU12 and MMAF was determined. Figure 2 provides the results. The CD19 ADC was formulated at pH 5, 6 and 7 and those formulations were tested over 14 days storage at 40°C. The most significant changes in the molecule were formation of high molecular weight (HMW) species, low molecular weight species (LMW), acidic variants (AV) and basic variants (BV). Figure 2 shows the percent change from initial for each degradant species following fourteen days at 40°C. Figure 2 shows that some degradants grew faster at high pH while others grew faster at low pH. Overall, the CD19 ADC comprising hBU12 and MMAF was most stable at pH 6.0.

### Example 2: Development of liquid and lyophilized formulations

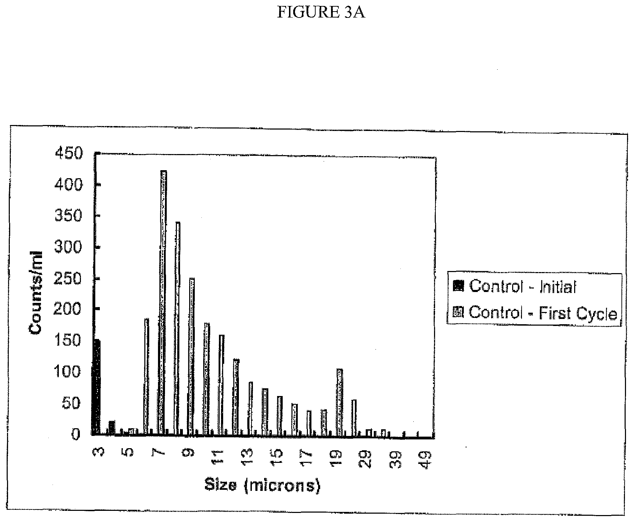

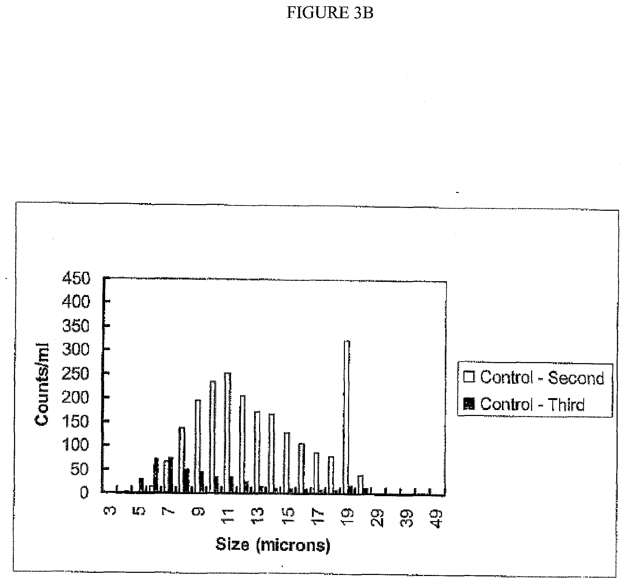

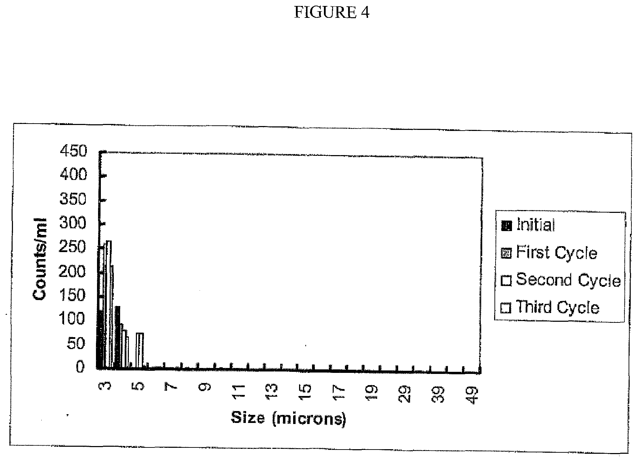

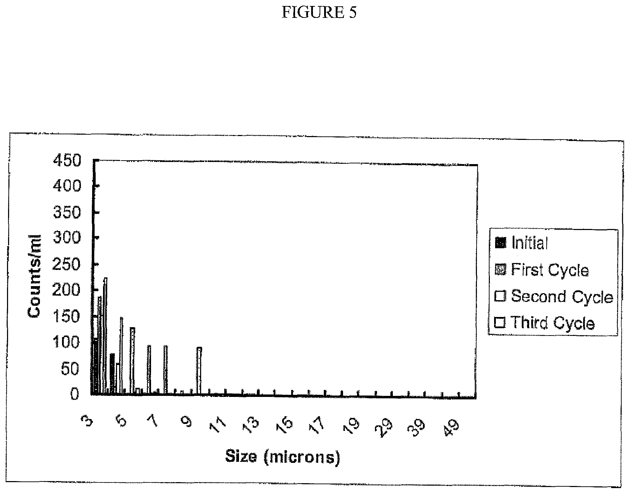

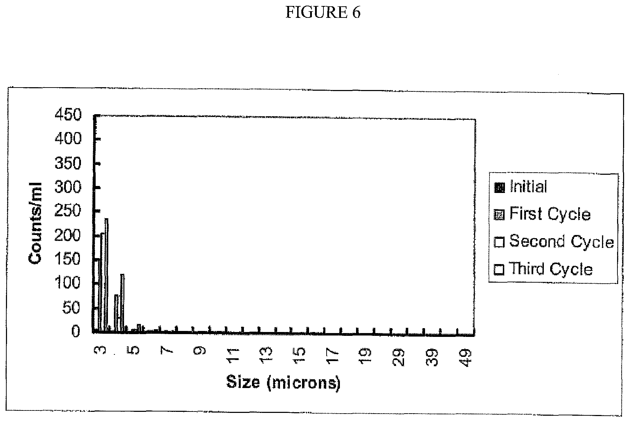

The effect of polysorbate 80 on the physical stability of hBU12 formulation was studied. Figures 3-6 show that the addition of polysorbate 80 improved the physical stability of hBU12 during freeze/thaw stress. Figures 3A and 3B indicate that the size and number of subvisible particles were increased significantly in hBU12 formulation during freeze/thaw in the absence of polysorbate 80. Figures 4-6 show that addition of polysorbate 80 at levels of 0.01%, 0.02% and 0.03% significantly reduced the amount of subvisible particles formed under the same conditions of freeze/thaw stress.

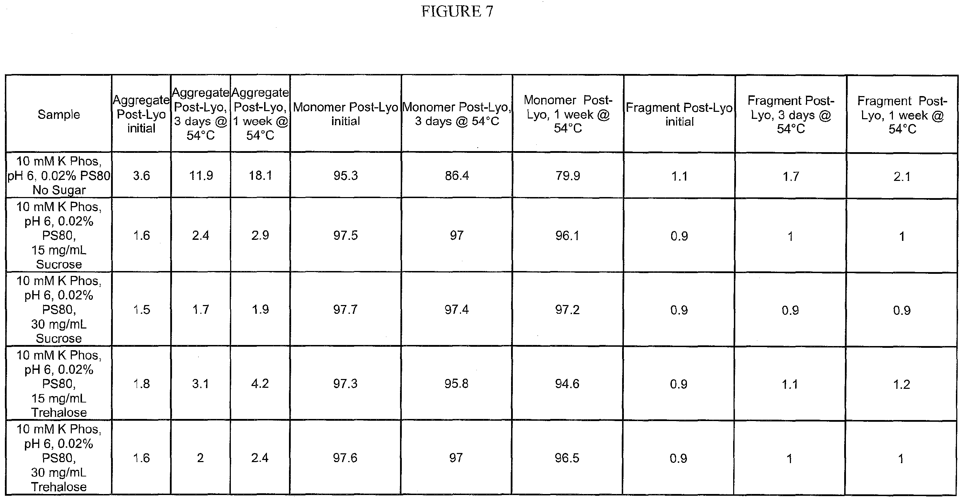

The effect of sugars on stability of a lyophilized formulation of a CD19 ADC comprising hBU12 and MMAF was assessed. Figure 7 summarizes data on the effect of two sugars, sucrose and trehalose, on the stability of CD19 ADC lyophilized formulations. The data indicates that both sugars impart stability on the CD19 ADC. Higher levels of both sugars impart greater stability and sucrose is more effective than trehalose at the same level.

Tables 5 and 6 demonstrate the effects of sucrose on aggregate and fragment formation in the lyophilized formulation of a CD19 ADC comprising hBU12 and MMAF over a two week period under stressed conditions, i.e., at 54°C. Lowest levels of aggregates and fragments (greatest stability) were seen after two weeks in the presence of 60mg/ml (6% w/v) sucrose.

**Table 5: Effect of sucrose on lyophilization stability - measurement of aggregates**

| **Sample** | **Aggregate Pre-Lyo initial** | **Aggregate Post-Lyo initial** | **Aggregate Post-Lyo, 3 days @ 54°C** | **Aggregate Post-Lyo, 1 week @ 54°C** | **Aggregate Post-Lyo, 2 weeks @ 54°C** |
|---|---|---|---|---|---|
| PDN-290-02-68, 30 mg/mL Sucrose | 1.3 | 1.3 | 1.6 | 1.7 | 2.1 |
| PDN-290-02-68, 45 mg/mL Sucrose | 1.3 | 1.3 | 1.5 | 1.5 | 1.7 |
| PDN-290-02-68, 60 mg/mL Sucrose | 1.3 | 1.3 | 1.4 | 1.5 | 1.6 |

**Table 6: Effect of sucrose on lyophilization stability - measurement of fragments**

| **Sample** | **Fragment Pre-Lyo initial** | **Fragment Post-Lyo initial** | **Fragment Post-Lyo, 3 days @ 54°C** | **Fragment Post-Lyo, 1 week @ 54°C** | **Fragment Post-Lyo, 2 weeks @ 54°C** |
|---|---|---|---|---|---|
| PDN-290-02-68, 30 mg/mL Sucrose | 0.8 | 0.8 | 0.9 | 1.0 | 1.0 |
| PDN-290-02-68, 45 mg/mL Sucrose | 0.8 | 0.8 | 0.9 | 0.9 | 0.9 |
| PDN-290-02-68, 60 mg/mL Sucrose | 0.8 | 0.8 | 0.9 | 0.9 | 0.9 |

Based on the results of development studies, a formulated solution of CD19A ADC at 15 mg/mL in 10 mM phosphate, 6% w/v sucrose, 0.02% w/v polysorbate 80 was selected for further development. The stability of this formulation has been examined in both the liquid and lyophilized state. For the liquid drug product, the formulated solution is filled directly into vials and stored as a liquid for long-term storage. For the lyophilized drug product, the formulated solution is filled into vials and lyophilized to produce a solid product for long-term storage.

The long-term stability of the liquid drug product is excellent as shown in Table 7. Most product quality attributes of the liquid drug product do not change significantly following 18 or 36 months of storage at 5°C. In particular, the biological assays and drug distribution on the ADC do not change. Attributes that do change slightly are high molecular weight (HMW) species, low molecular weight (LMW) species and acidic variants (AV). The changes in these attributes from initial following 18 months storage at 5°C for two liquid drug product lots are summarized in Table 7, as are the results at 36 months for drug lot DEVGLY-1.

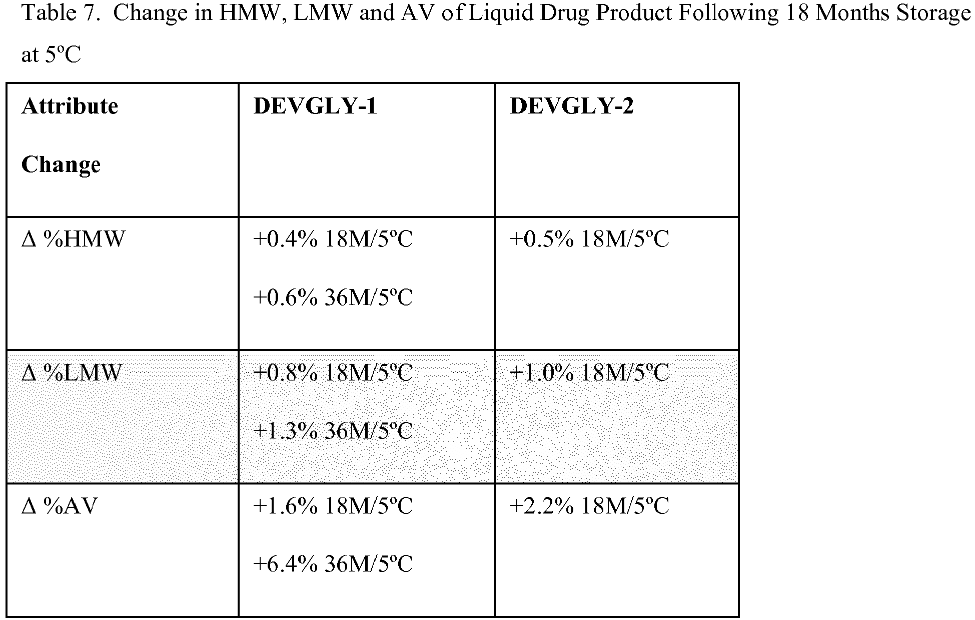

The long-term stability of the lyophilized drug product is excellent as shown in Table 8. Most product quality attributes of the lyophilized drug product do not change significantly following 18, 30 or 36 months of storage at 25°C and 12 months of storage at 40°C. In particular, the biological assays and drug product distribution on the ADC do not change. Stress storage at 40°C shows slight change in some product attributes including high molecular weight (HMW) species, low molecular weight (LMW) species and basic variants (BV). At 25°C long-term storage there is a slight increase in HMW and no other changes. The changes in these attributes from initial following 18, 30 or 36 months storage at 25°C and 12 months of storage at 40°C are summarized in Table 8.

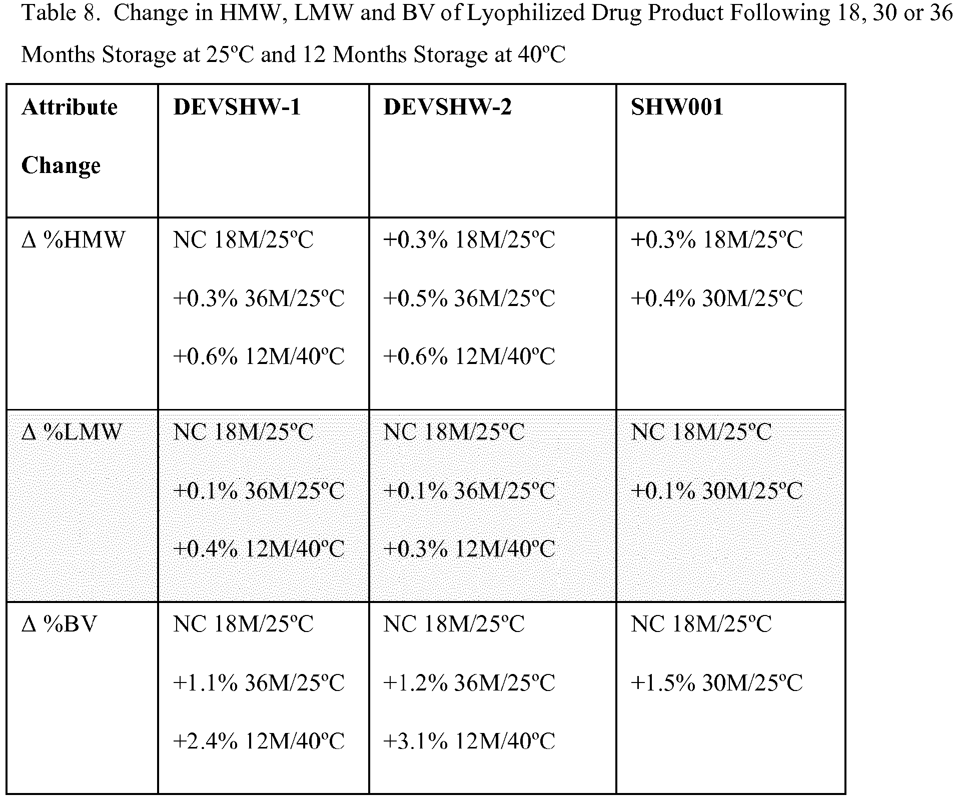

It is understood that the examples and embodiments described herein are for illustrative purposes only and that various modifications or changes in light thereof will be suggested to persons skilled in the art and are to be included within the scope of the appended claims.

## Claims

1. A stable formulation of 15 mg/ml of an anti-CD 19 antibody conjugated to monomethylauristatin F (MMAF), in 10mM potassium phosphate buffer, wherein the anti-CD19 antibody is a humanized IgG1 antibody comprising a full length light chain having a variable region of SEQ ID NO:1 and a full length heavy chain having a variable region of SEQ ID NO:2, wherein the anti-CD 19 antibody is conjugated to maleimidocaproyl monomethyl auristatin F,
the formulation comprising a surfactant and a bulking agent, wherein the potassium phosphate buffer has a pH value between 5.0 and 7.0, wherein the surfactant is polysorbate 80 at a concentration 0.02% w/v, and wherein the bulking agent is sucrose at a concentration of 6% w/v.

2. The stable formulation of claim 1, wherein the potassium phosphate buffer has a pH between 5.5 and 6.5.

3. The stable formulation of claim 1, wherein the potassium phosphate buffer has a pH of 6.0.

4. A stable formulation of claim 1, wherein the potassium phosphate buffer is a potassium phosphate buffer at pH 6.0.

5. A stable lyophilized anti-CD 19 antibody formulation, made by lyophilizing the formulation of claim 4.

6. A vial comprising the stable lyophilized anti-CD 19 antibody formulation of claim 5 or the stable anti-CD19 antibody formulation of claim 4 in an amount for administration to a patient in need of such formulation.

## Patentansprüche

1. Stabile Formulierung von 15 mg/ml eines Anti-CD19-Antikörpers, der mit Monomethylauristatin F (MMAF) konjugiert ist, in 10 mM Kaliumphosphatpuffer, wobei der Anti-CD19-Antikörper ein humanisierter IgG1-Antikörper ist, der eine leichte Kette voller Länge mit einem variablen Bereich von SEQ ID NO: 1 und eine schwere Kette voller Länge mit einem variablen Bereich von SEQ ID NO: 2 umfasst, wobei der Anti-CD19-Antikörper an Maleimidocaproylmonomethylauristatin F konjugiert ist, wobei die Formulierung ein oberflächenaktives Mittel und ein Füllmittel umfasst, wobei der Kaliumphosphatpuffer einen pH-Wert zwischen 5,0 und 7,0 aufweist, wobei das oberflächenaktive Mittel Polysorbat 80 in einer Konzentration von 0,02 % (Gew./Vol.) ist und wobei das Füllmittel Saccharose in einer Konzentration von 6 % (Gew./Vol.) ist.

2. Stabile Formulierung nach Anspruch 1, wobei der Kaliumphosphatpuffer einen pH-Wert zwischen 5,5 und 6,5 aufweist.

3. Stabile Formulierung nach Anspruch 1, wobei der Kaliumphosphatpuffer einen pH-Wert von 6,0 aufweist.

4. Stabile Formulierung nach Anspruch 1, wobei der Kaliumphosphatpuffer ein Kaliumphosphatpuffer mit einem pH-Wert von 6,0 ist.

5. Stabile lyophilisierte Anti-CD19-Antikörper-Formulierung, hergestellt durch Lyophilisieren der Formulierung nach Anspruch 4.

6. Fläschchen, umfassend die stabile lyophilisierte Anti-CD19-Antikörperformulierung nach Anspruch 5 oder die stabile Anti-CD19-Antikörperformulierung nach Anspruch 4 in einer Menge zur Verabreichung an einen Patienten, der eine solche Formulierung benötigt.

## Revendications

1. Formulation stable de 15 mg/ml d'un anticorps anti-CD19 conjugué à la monométhylauristatine F (MMAF), dans un tampon de phosphate de potassium 10mM, dans laquelle l'anticorps anti-CD19 est un anticorps IgG1 humanisé comprenant une chaîne légère pleine longueur comportant une région variable de SEQ ID N° : 1 et une chaîne lourde pleine longueur comportant une région variable de SEQ ID N° : 2, dans laquelle l'anticorps anti-CD19 est conjugué au maléimidocaproyl monométhyl auristatine F, la formulation comprenant un tensioactif et un agent gonflant, dans laquelle le tampon phosphate de potassium a un pH compris entre 5,0 et 7,0, dans laquelle le tensioactif est le polysorbate 80 à une concentration de 0,02 % p/v, et dans laquelle l'agent gonflant est le saccharose à une concentration de 6 % p/v.

2. Formulation stable selon la revendication 1, dans laquelle le tampon phosphate de potassium a un pH compris entre 5,5 et 6,5.

3. Formulation stable selon la revendication 1, dans laquelle le tampon phosphate de potassium a un pH de 6,0.

4. Formulation stable selon la revendication 1, dans laquelle le tampon phosphate de potassium est un tampon phosphate de potassium à un pH de 6,0.

5. Formulation d'anticorps anti-CD19 lyophilisée stable, fabriquée par la lyophilisation de la formulation de la revendication 4.

6. Flacon comprenant la formulation d'anticorps anti-CD19 lyophilisée stable selon la revendication 5 ou la formulation d'anticorps anti-CD19 stable selon la revendication 4 en une quantité destinée à être administrée à un patient ayant besoin d'une telle formulation.


### SEQUENCE LISTING

<110> SEATTLE GENETICS, INC.
<120> STABLE FORMULATIONS FOR ANTI-CD19 ANTIBODIES AND ANTIBODY-DRUG CONJUGATES
<130> 0019-00311PC
<150> US 61/976,313
   <151> 2014-04-07
<160> 2
<170> PatentIn version 3.5
<210> 1
   <211> 107
   <212> PRT
   <213> Artificial
<220>
   <223> hBU12 light chain variable region
<400> 1
   Glu Ile Val Leu Thr Gln Ser Pro Ala Thr Leu Ser Leu Ser Pro Gly
   1               5                   10                  15
   Glu Arg Ala Thr Leu Ser Cys Ser Ala Ser Ser Ser Val Ser Tyr Met
               20                  25                  30
   His Trp Tyr Gln Gln Lys Pro Gly Gln Ala Pro Arg Leu Leu Ile Tyr
           35                  40                  45
   Asp Thr Ser Lys Leu Ala Ser Gly Ile Pro Ala Arg Phe Ser Gly Ser
       50                  55                  60
   Gly Ser Gly Thr Asp Phe Thr Leu Thr Ile Ser Ser Leu Glu Pro Glu
   65                  70                  75                  80
   Asp Val Ala Val Tyr Tyr Cys Phe Gln Gly Ser Val Tyr Pro Phe Thr
                   85                  90                  95
   Phe Gly Gln Gly Thr Lys Leu Glu Ile Lys Arg
               100                 105
<210> 2
   <211> 120
   <212> PRT
   <213> Artificial
<220>
   <223> hBU12 heavy chain variable region
<400> 2
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Gly Ser Ile Ser Thr Ser
               20                  25                  30
   Gly Met Gly Val Gly Trp Ile Arg Gln His Pro Gly Lys Gly Leu Glu
           35                  40                  45
   Trp Ile Gly His Ile Trp Trp Asp Asp Asp Lys Arg Tyr Asn Pro Ala
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Met Glu Leu Trp Ser Tyr Tyr Phe Asp Tyr Trp Gly Gln
               100                 105                 110
   Gly Thr Leu Val Thr Val Ser Ser
           115                 120